# EUROPEAN PATENT APPLICATION

(11) **EP 1 637 630 A1**
(43) Date of publication of application: **22.03.2006**
(21) Application number: 04730020.7
(22) Date of filing: 28.04.2004
(51) Int. Cl.: C30B 29/58

(54) **APPARATUS FOR SCREENING PROPTEIN CRYSTALLIZATION CONDITIONS**

(30) Priority: 28.04.2003 JP 2003124085
(71) Applicant: MATSUSHITA ELECTRIC INDUSTRIAL CO., LTD., Kadoma-shi, Osaka 571-8501 (JP)
(72) Inventor: MOTEGI, Itsuro, Fukuoka 811-4162 (JP); MATSUZAKI, Hirofumi, Fukuoka 815-0073 (JP); KITAHARA, Hideyoshi, Fukuoka- 816-0874 (JP); TSUKAMOTO, Mitsuhaya, Fukuoka 811-1351- (JP); SHIMOGAWA, Kouji, Fukuoka 830-0062 (JP); MAGOORI, Masataka, Saga 849-0918 (JP); HIGUCHI, Akira, Fukuoka 839-0853 (JP)
(74) Representative: Vossius & Partner
(86) International application number: PCT/JP2004/006147
(87) International publication number: WO 2004/097082

(57) **Abstract**

The present invention is intended to provide an apparatus for screening protein crystallization conditions that can screen protein crystallization conditions efficiently by the vapor diffusion method using the sitting drop technique. In order to achieve the above-mentioned object, in an apparatus for screening protein crystallization conditions that screens protein crystallization conditions using the sitting drop technique that is one of the techniques of protein crystallization to be carried out by the vapor diffusion method, an apparatus 2 for preparing a crystallization plate that includes a dispensing means for dispensing a protein solution and a crystallization solution in wells of the crystallization plate and a seal attachment unit that seals the wells that have been subjected to dispensation is connected to a protein crystal detection apparatus 5 that detects protein crystals produced in the crystallization plate in a thermostatic chamber that stores the crystallization plate that has been subjected to the dispensing in a predetermined environment. Accordingly, the crystallization plate is transferred automatically and thus screening is carried out efficiently and automatically.

## Description

### Technical Field

The present invention relates to an apparatus for screening protein crystallization conditions that screens conditions for crystallizing protein contained in a protein solution. The apparatus of the present invention can be used not only for screening protein crystallization conditions but also for producing protein crystals, for example.

### Background Art

Recently, studies have been undertaken actively to use genetic information effectively in a field of medical treatments, etc., and efforts are being made to analyze the structure of protein that composes a gene, which is to be used as the basic technology in the studies. This analysis of the protein structure is intended to determine a three-dimensional structure formed of amino acids composing the protein that are sequenced in the form of a three-dimensional line. The analysis is carried out by a method such as X-ray crystal structure analysis, for example.

In order to carry out such an analysis of the protein structure, first it is necessary to crystallize the protein that is an object to be analyzed. A vapor diffusion method has been known as the method of crystallizing protein. In this method, a solvent component that evaporates from a protein solution containing protein to be crystallized is allowed to be absorbed by a crystallization solution contained in the same container. This allows the protein solution to be maintained in a supersaturation state and thereby crystals are generated gradually.

In order to crystallize protein by the vapor diffusion method, a hanging drop technique and a sitting drop technique can be used. In the hanging drop technique, a solvent is evaporated in a hanging state where a drop of a protein solution is deposited and kept on the lower surface of a solution holding surface. In the sitting drop technique, a solvent is evaporated in a seating state where a drop of a protein solution is deposited and kept on the upper surface of a solution holding part. The protein crystallization to be carried out by such vapor diffusion methods requires a complicated test operation. Hence, conventionally, containers (see, for instance, JP2002-179500A) and automation apparatuses (see, for instance, JP2003-14596A have been proposed that are designed specifically to carry out experiments efficiently.

### Disclosure of Invention

The conventional technique concerning the above-mentioned automation apparatus, however, can be applied only to the hanging drop technique. In the conventional technique, the scheme of automation has not been established with respect to the vapor diffusion method using the sitting drop technique. Conventionally, it therefore was difficult to carry out the screening of protein crystallization conditions efficiently by the vapor diffusion method using the sitting drop technique.

It therefore is an object of the present invention to provide an apparatus for screening protein crystallization conditions that allows protein crystallization conditions to be screened efficiently by the vapor diffusion method using the sitting drop technique.

In order to achieve the above-mentioned object, the apparatus for screening protein crystallization conditions of the present invention screens protein crystallization conditions to be employed in the sitting drop technique that is one of the techniques for protein crystallization to be carried out by the vapor diffusion method. The apparatus is characterized in including: a dispensing stage where a crystallization vessel provided with a plurality of solution storage parts is set, with each solution storage part including a solution holding part that holds a protein solution in a seating state from the lower side and a reservoir that retains a crystallization solution; a dispensing means that dispenses the crystallization solution in the reservoir and dispenses the protein solution in the solution holding part, in the solution storage parts of the crystallization vessel set on the dispensing stage; a sealing means that seals the solution storage parts in which the crystallization solution and the protein solution have been dispensed; a crystallization vessel storage means that stores a plurality of crystallization vessels in a predetermined environment, with each of the crystallization vessels whose solution storage parts have been sealed; a protein crystal detection means that detects protein crystals generated in the protein solution contained in the solution storage parts that have been sealed; and a crystallization vessel transfer means that transfers the crystallization vessels to at least one of the dispensing stage, the sealing means, the crystallization vessel storage means, and the protein crystal detection means.

The screening apparatus of the present invention allows protein crystallization conditions to be screened efficiently by the vapor diffusion method using the sitting drop technique. Furthermore, the apparatus of the present invention can be used not only for screening the protein crystallization conditions but also for producing protein crystals, for example.

### Brief Description of Drawings

FIG. 1 is a perspective view showing an apparatus for screening protein crystallization conditions according to an embodiment of the present invention.
FIG. 2 is a perspective view showing an apparatus for preparing a protein crystallization plate according to an embodiment of the present invention.
FIG. 3 is a perspective view showing a crystallization plate to be used in an embodiment of the present invention.
FIG. 4 is a partial cross-sectional view showing the crystallization plate to be used in an embodiment of the present invention.
FIG. 5 is an elevation view showing a dispensing head part of an apparatus for preparing a protein crystallization plate according to an embodiment of the present invention.
FIGs. 6A, 6B, and 6C are drawings for explaining a dispensing operation that is carried out in the apparatus for preparing a protein crystallization plate according to an embodiment of the present invention.
FIG. 7 is a block diagram showing the configuration of a control system of the apparatus for preparing a protein crystallization plate according to an embodiment of the present invention.
FIG. 8 is a flow chart showing an operation for preparing a protein crystallization vessel by the apparatus for preparing a protein crystallization plate according to an embodiment of the present invention.
FIG. 9 is a perspective view showing a protein crystal detection apparatus according to an embodiment of the present invention.
FIG. 10 is a cross-sectional view showing the protein crystal detection apparatus according to an embodiment of the present invention.
FIG. 11 is a partial cross-sectional view showing an observation section of the protein crystal detection apparatus according to an embodiment of the present invention.
FIG. 12 is a block diagram showing the configuration of a control system of the protein crystal detection apparatus according to an embodiment of the present invention.
FIG. 13 is a flow chart showing an observation operation to be carried out by the protein crystal detection apparatus according to an embodiment of the present invention.
FIG. 14 is a flow chart showing a protein crystal detection process to be carried out by the protein crystal detection apparatus according to an embodiment of the present invention.

### Description of the Invention

In the screening apparatus of the present invention, it is preferable that the dispensing means include: a crystallization solution dispensing head that dispenses the crystallization solution; and a protein solution dispensing head that dispenses the protein solution.

In the screening apparatus of the present invention, it is preferable that the crystallization solution dispensing head include: a first dispensing head that dispenses the crystallization solution in the reservoir; and a second dispensing head that dispenses the crystallization solution in the solution holding part.

In the screening apparatus of the present invention, it is preferable that the second dispensing head draw in the crystallization solution from the reservoir and then dispense it in the solution holding part.

In the screening apparatus of the present invention, the dispensing means may include: a single dispensing head part including the crystallization solution dispensing head and the protein solution dispensing head; and a dispensing-head moving means that moves the dispensing head part with respect to the dispensing stage.

In the screening apparatus of the present invention, it is preferable that the dispensing means include: a single dispensing head part including the first dispensing head, the second dispensing head, and the protein solution dispensing head; and a dispensing-head moving means that moves the dispensing head part with respect to the dispensing stage.

In the screening apparatus of the present invention, it is preferable that the crystallization vessel transfer means include: a first transfer means that transfers the crystallization vessel to at least one of the dispensing stage and the sealing means; and a second transfer means that transfers the crystallization vessel to a crystallization solution storage means.

In the screening apparatus of the present invention, it is preferable that the crystallization vessel storage means include: a thermostatic chamber in which the crystallization vessel is stored in the predetermined environment; and a protein crystal detection means that detects crystals of protein generated in the crystallization vessel placed in the thermostatic chamber, and the second transfer means transfer the crystallization vessel within the thermostatic chamber.

A computer program of the present invention allows an operation for preparing a protein crystallization plate to be carried out in the screening apparatus of the present invention that is controlled by a computer. The computer program allows the apparatus to carry out, through the computer: a step of transferring the crystallization plate that is empty to the dispensing stage with the transfer means; a step of reading dispensation information; a step of carrying out dispensation in a well of the plate with the dispensing means according to the information; a step of identifying the next well to be subjected to dispensation; a step of transferring the plate subjected to the dispensation to the sealing means with the transfer means; and a step of sealing the plate with the sealing means.

Preferably, the computer program of the present invention also allows the apparatus to carry out a step of recording the information about the protein crystallization plate prepared as described above in the computer.

Another computer program of the present invention allows protein crystals generated in a protein crystallization plate to be observed in the screening apparatus of the present invention that is controlled by a computer. The computer program allows the apparatus to carry out, through the computer: a step of transferring the crystallization plate to the observation stage by the transfer means; a step of positioning the first well of the plate in a position where observation is to be made; a step of capturing an image of the inside of the well; a step of detecting protein crystals based on the image; a step of identifying the next well to be observed and transferring the well to the position where observation is to be made; and a step of recording the observation results in the computer.

In the computer program of the present invention, it is preferable that the step of detecting protein crystals based on the image include: a step of processing the image; a step of judging whether crystallization has occurred, which is carried out based on the processed image; and a step of recording at least one selected from information about the plate, information about the well, the image, and the observation time in the computer.

The apparatus for screening protein crystallization conditions of the present invention can be controlled with a computer and preferably is controlled by a computer program of the present invention.

The apparatus for screening protein crystallization conditions of the present invention can be used as an apparatus for producing protein crystals.

Next, an example of embodiments according to the present invention is described using the drawings.

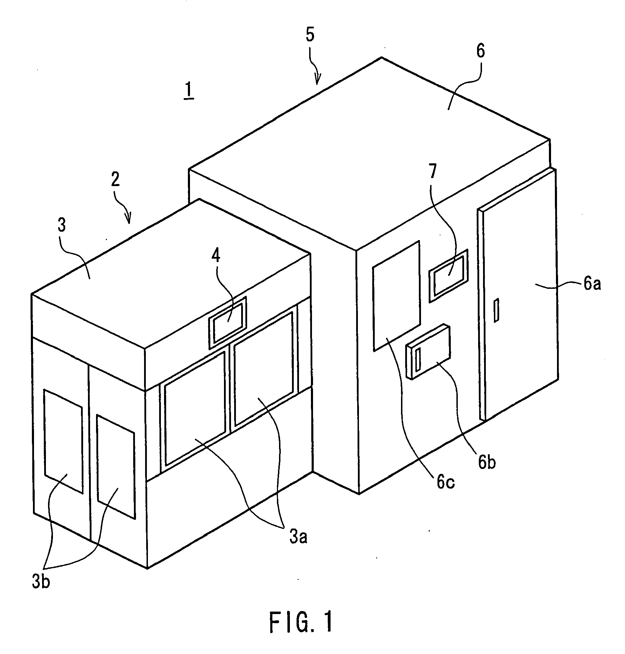

First, the overall configuration of an example of the apparatus for screening protein crystallization conditions of the present invention is described with reference to FIG. 1. The apparatus 1 for screening protein crystallization conditions screens protein crystallization conditions to be employed in the sitting drop technique that is one of the techniques of protein crystallization to be carried out by a vapor diffusion method. The apparatus is configured with an apparatus 2 for preparing a protein crystallization plate and a protein crystal detection apparatus 5 that are individual apparatuses, respectively, and are joined together.

The apparatus 2 for preparing a protein crystallization plate (an apparatus for preparing a crystallization vessel) carries out the dispensing operation with respect to the crystallization plate that is a crystallization vessel and thereby performs a process for preparing a crystallization plate. In this process, predetermined crystallization conditions are set. The protein crystal detection apparatus 5 stores a plurality of crystallization vessels thus prepared, in a predetermined environment and then carries out protein crystal detection with respect to these crystallization vessels.

The apparatus 2 for preparing a protein crystallization plate and the protein crystal detection apparatus 5 are configured, with the respective functional parts to be described later being contained inside box-shaped housings 3 and 6, respectively. The housing 3 is provided with: windows 3a for observation and access to the inside thereof; part supply doors 3b for supplying consumable parts; and a control panel 4. The housing 6 is configured to be a thermostatic chamber whose inner environment temperature is maintained at a predetermined temperature. The housing 6 is provided with: a door 6a for access to the inside thereof; a small door 6b for the operation/check to be performed from the front side thereof; a window 6b for checking the inside thereof; and a control panel 7. As described later, an opening for transferring a vessel is provided of the boundary between the housings 3 and 6, so that a crystallization plate prepared in the apparatus 2 for preparing a protein crystallization plate can be transferred directly into the protein crystal detection apparatus 5.

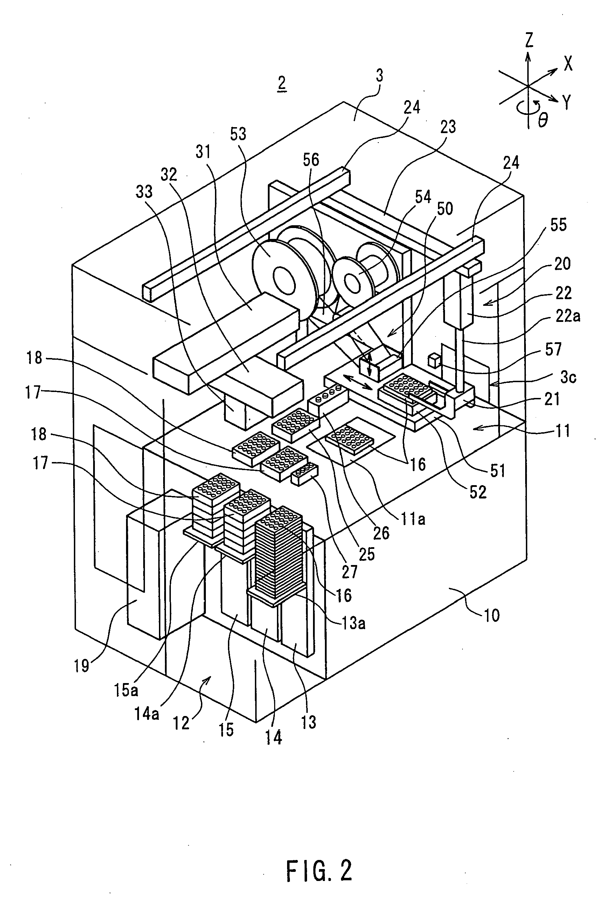

Next, an example of the internal configuration of the apparatus 2 for preparing a protein crystallization plate is described with reference to FIG. 2. The upper surface of a platform 10 provided inside the apparatus 2 for preparing a protein crystallization plate serves as an operation area 11 where various processing and operations are carried out with respect to the crystallization plates. The side face of the platform 10 located on the left side of the drawing is provided with a stock section 12 where consumable supplies are stored. The stock section 12 is provided with a plate stock part 13 and two rack stock parts 14 and 15.

The plate stock part 13 is equipped with a lifting plate 13a on which microplates 16 for crystallization (hereinafter referred to simply as a "crystallization plate 16") are stacked in multiple stages to be stored. When the lifting plate 13a goes up, stacked crystallization plates 16 goes up and the crystallization plate 16 located at the top is taken out by a transfer part 20 to be described later. The crystallization plate 16 is a crystallization vessel to be used for crystallizing protein contained in a protein solution.

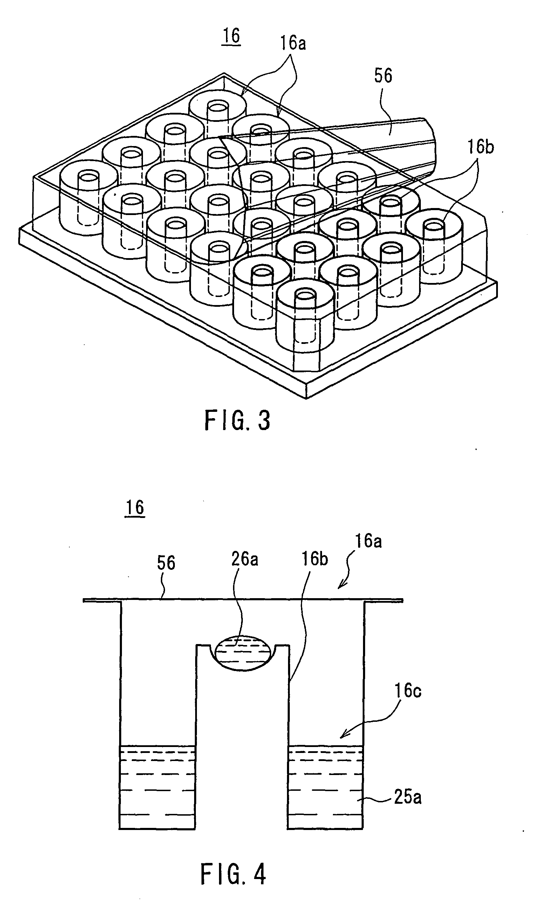

Now, an example of the crystallization plate 16 is described with reference to FIGs. 3 and 4. As shown in FIG. 3, the crystallization plate 16 includes a plurality of wells 16a arranged in the form of a lattice. The wells 16a are so-called caldera-like concave parts for containing a liquid, each of which is provided with a cylindrical liquid holding part 16b at the center of a circular concave part. A sample to be crystallized, i.e. a protein solution 26a containing protein to be crystallized, and a crystallization solution 25a to be used for crystallization are dispensed in the wells 16a. The size of the crystallization plate 16 is not particularly limited but can be a standardized size, for example. Examples of the standard include the SBS standard, etc. The size of the wells 16a also is not particularly limited but they can have a diameter of 10 mm to 20 mm, for example. Furthermore, the size of the liquid holding part 16b is not particularly limited, but it can have, for instance, a diameter that is half the diameter of the wells 16a.

FIG. 4 shows an example of the section of one well 16a containing such a sample. In the well 16a, a drop of the protein solution 26a is seated to be held in a pocket provided in the top part of the liquid holding part 16b. A crystallization solution 25a is stored in a ring-shaped reservoir 16c surrounding the liquid holding part 16b. The well 16a is a solution storage part including: a liquid holding part 16b that allows the protein solution that is subjected to crystallization to be held in a seating state from the lower side; and the reservoir 16c that stores the crystallization solution 25a. As described later, when crystallization is to be started, a predetermined amount of crystallization solution 25a is taken from the reservoir 16c to be dispensed into the protein solution 26a held by the liquid holding part 16b, which then are mixed together. Thereafter, a seal member 56 is attached to the upper surfaces of the respective wells 16a (see FIG. 3).

The crystallization plate 16 is stored in this state in a predetermined temperature atmosphere and thereby a solvent component contained in the protein solution 26a is evaporated. Accordingly, the protein concentration of the protein solution 26a increases, which brings the protein solution 26a into a supersaturation state to produce protein crystals. In this case, the evaporation of the solvent from the protein solution 26a progresses gradually, with the solvent that evaporates from the protein solution 26a being kept in equilibrium with the vapor to be absorbed by the crystallization solution 25a. Thus, crystals are produced stably.

The rack stock parts 14 and 15 shown in FIG. 2 are equipped with lifting plates 14a and 15a, respectively, on which tip racks 17 and 18 are stacked to be stored like the crystallization plates 16. The tip racks 17 and 18 are taken out by the transfer part 20. The tip racks 17 and 18 store a plurality of disposable dispensing tips to be used for dispensing the crystallization solution 25a during the dispensing operation, with the dispensing tips being arranged in the form of a lattice. For the dispensing of the crystallization solution 25a, two types of dispensing tips, specifically large and small dispensing tips, are used as described later. The tip racks 17 and 18 store small-sized and large-sized tips for dispensing the crystallization solution, respectively.

These crystallization plate 16 and tip racks 17 and 18 are transferred, by the transfer part 20 to be described later, to the operation area 11 where the dispensing tips taken out from the tip racks 17 and 18 are used for the dispensing operation. The dispensing tips thus used are returned to the tip racks 17 and 18. The stock section 12 is equipped with a disposal box 19 for collecting the consumable parts that have been used. The tip racks 17 and 18 containing the dispensing tips that have been used are thrown away into the disposal box 19 by the transfer part 20.

The transfer part 20 is described now. Two rows of X-axis mechanisms 24 are disposed above the platform 10 in the X direction while a Z*θ* axis mechanism 22 is attached to a Y-axis mechanism 23 disposed across the X-axis mechanisms 24. A transfer head 21 is joined to an axis part 22a that extends downward from the Z*θ* axis mechanism 22. The transfer head 21 moves in the X, Y, and Z*θ* directions in the operation area 11 by driving the X-axis mechanism 24, the Y-axis mechanism 23, and the Z*θ* axis mechanism 22, and clamps and transfers the crystallization plate 16 and tip racks 17 and 18.

Next, the operation area 11 is described. A dispensing stage 11a is provided in the substantial center of the operation area 11. The crystallization plate 16 taken out from the stock section 12 is set on the dispensing stage 11a. The tip racks 17 and 18 as well as a nozzle rack 27 for storing nozzles for dispensing the protein solution to be described later are placed in the area between the dispensing stage 11a and the stock section 12. Furthermore, a protein solution supply reservoir 26 that stores a protein solution 26a to be screened and a crystallization solution supply reservoir 25 that stores a crystallization solution 25a to be used for crystallization are placed in the area located behind the dispensing stage 11a.

The crystallization plate preparation process for preparing crystallization plates to be subjected to the test in screening is carried out as follows. That is, the protein solution 26a drawn from the protein solution supply reservoir 26 and the crystallization solution 25a drawn from the crystallization solution supply reservoir 25 are dispensed in an empty crystallization plate 16 using the dispensing means to be described below.

An X-axis table 31 is disposed in the X-direction above the operation area 11 including the dispensing stage 11a. A dispensing head part 33 is attached to a Y-axis table 32 joined to the X-axis table 31. The X-axis table 31 and the Y-axis table 32 are driven to move the dispensing head part 33 above the operation area 11 including the dispensing stage 11a.

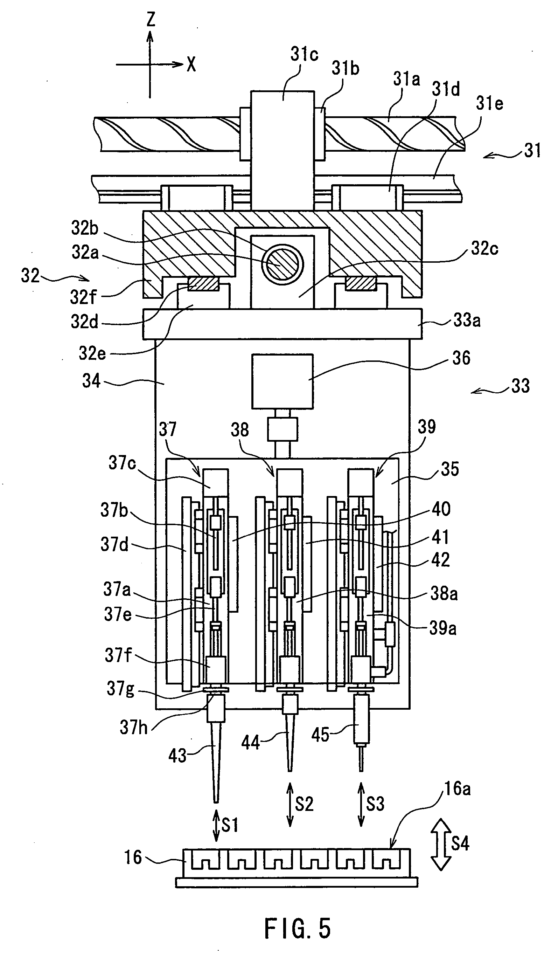

FIG. 5 shows an example of dispensing-head moving mechanism. As shown in FIG. 5, the X-axis table 31 is configured to drive a movable block 32f that is guided by a guide mechanism composed of an X guide 31e and a slider 31d, in the X direction through a block 31c by a direct-acting mechanism composed of a feed screw 31a and a nut 31b. The Y axis table 32 is configured to drive a movable plate 33a that is guided by a guide mechanism composed of a Y guide 32e and a slider 32d, in the Y direction through a block 32c by a direct-acting mechanism composed of a feed screw 32a and a nut 32b.

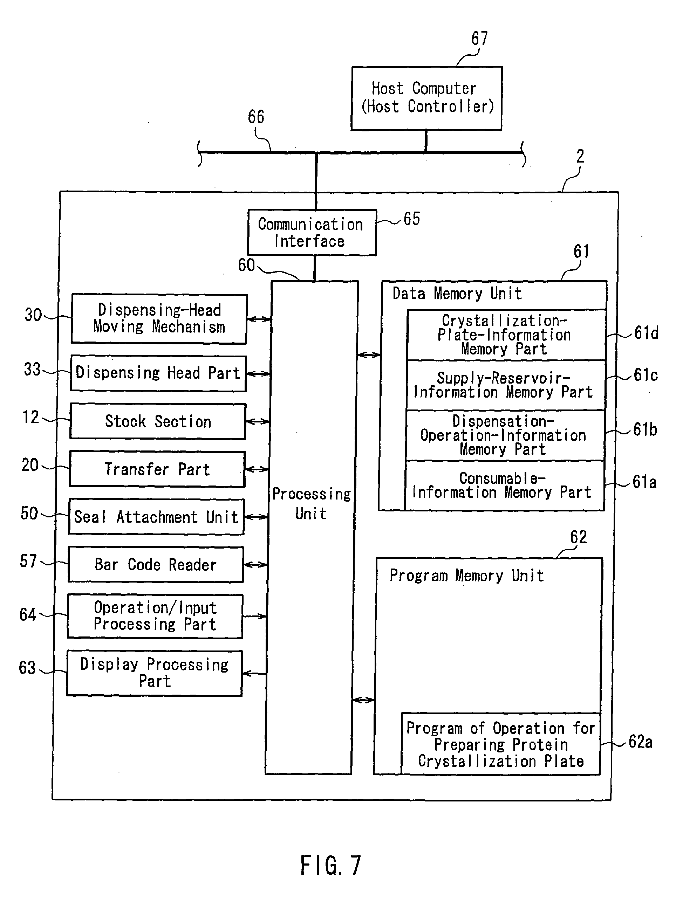

Next, the description is directed to the dispensing head part 33. A perpendicular dispensing head base member 34 is attached to the lower face of the movable plate 33a. A lifting plate 35 is attached to the dispensing head base member 34 so as to be slidable in the Z direction. The lifting plate 35 is moved up and down by a motor 36 for moving it up and down that is fixed to the dispensing head base member 34. The dispensing head base member 34 and the motor 36 compose a Z-axis table. This Z-axis table, the X-axis table 31, and the Y-axis table 32 compose a dispensing-head moving mechanism 30 (see FIG. 7) that moves the dispensing head part 33.

The lifting plate 35 is provided with three dispensing heads, specifically, a first dispensing head 37, a second dispensing head 38, and a third dispensing head 39. Among them, the first dispensing head 37 and the second dispensing head 38 are both crystallization solution dispensing heads that are used for dispensing the crystallization solution 25a. The first dispensing head 37 is used for dispensing a large amount of crystallization solution 25a in a short period of time while the second dispensing head 38 is used for dispensing a small amount of crystallization solution 25a with high accuracy. The third dispensing head 39 is a protein solution dispensing head that is used for dispensing the protein solution 26a.

The configurations of these dispensing heads are described now. The first dispensing head 37, the second dispensing head 38, and the third dispensing head 39 are different from one another in their dispensing tips to be attached thereto, but are identical to one another with respect to their basic functions such as suction and discharge of a liquid. Hence, the following description is directed to the first dispensing head 37, and the descriptions of the parts of the second dispensing head 38 and the third dispensing head 39 that are common to those of the first dispensing head 37 are not repeated.

The first dispensing head 37 is provided for the lifting plate 35 and is configured with a first lifting member 37a that is provided to be slidable along a perpendicular guide 37d with respect to the lifting plate 35. The first lifting member 37a is moved up and down by a first head selection cylinder 40 by a predetermined stroke.

A lower part of the first lifting member 37a is provided with a cylinder part 37f into which a plunger 37e is inserted from the upper side. The plunger 37e is moved by a plunger lifting mechanism 37b provided with a motor 37c. The plunger 37e moves up and down in the cylinder part 37f and thereby the cylinder part 37f functions as a pump mechanism.

The lower part of the cylinder part 37f is joined to a nozzle 37h to which a first dispensing tip 43 is attached. The first dispensing tip 43 is a large-sized tip for dispensing a crystallization solution and is supplied from the tip rack 18. When the first dispensing tip 43 is to be attached, first the dispensing head part 33 is moved to a position above the tip rack 18 storing the first dispensing tip 43. Then the nozzle 37h is moved down to be inserted into an attachment opening provided at the upper end of the first dispensing tip 43.

The nozzle 37h is provided with a tip detachment plate 37g. When the tip detachment plate 37g is moved down, with the first dispensing tip 43 being attached to the nozzle 37h, the first dispensing tip 43 is detached from the nozzle 37h. In this manner, all the operations of attaching and detaching the first dispensing tip 43 with respect to the first dispensing head 37 can be carried out automatically.

The following descriptions are directed to the second dispensing head 38 and the third dispensing head 39. The second dispensing head 38 and the third dispensing head 39 are configured to move a second lifting member 38a and a third lifting member 39a up and down by a second head selection cylinder 41 and a third head selection cylinder 42, respectively. The second lifting member 38a and the third lifting member 39a are provided with the same mechanisms as the above-mentioned plunger lifting mechanism 37b and cylinder part 37f, respectively. A second dispensing tip 44 and a dispensing nozzle 45 are attached to the second dispensing head 38 and the third dispensing head 39, respectively.

The second dispensing tip 44 is a small-sized tip for dispensing a crystallization solution and is supplied from the tip rack 17. The dispensing nozzle 45 is a nozzle for dispensing a protein solution and is supplied from the nozzle rack 27. The operations of attaching and detaching the second dispensing tip 44 and the dispensing nozzle 45 also are the same as in the case of the first dispensing tip 43.

Next, the description is directed to a dispensing operation that is carried out with the dispensing head part 33. The first dispensing head 37, the second dispensing head 38, and the third dispensing head 39 are moved up and down by the first head selection cylinder 40, the second head selection cylinder 41, and the third head selection cylinder 42 by predetermined strokes S1, S2, and S3, respectively. Furthermore, the first dispensing head 37, the second dispensing head 38, and the third dispensing head 39 are moved up and down by a stroke S4 at the same time by the Z-axis table (the motor 36 for moving them up and down) that moves them up and down together.

When the dispensing operation is carried out, one of the above-mentioned three dispensing heads is selected according to the object and purpose in the dispensing operation concerned. The head selection cylinder corresponding to the dispensing head thus selected is driven to move down the selected dispensing head alone and thereby allows the lower end of the corresponding dispensing tip or dispensing nozzle to protrude downward further than that of the dispensing tip or dispensing nozzle that has not been selected. For example, when the first dispensing head 37 is selected, the first dispensing tip 43 moves down by the stroke S1. Similarly, when the second dispensing head 38 or the third dispensing head 39 is selected, the second dispensing tip 44 or the dispensing nozzle 45 moves down by the stroke S2 or S3, respectively. These strokes S1, S2, and S3 are set individually according to the height of the part in which dispensing is to be carried out.

Then the lifting plate 35 is allowed to move down by the stroke S4, with any one of the dispensing heads having been moved down. Thus, any one of the first dispensing tip 43, the second dispensing tip 44, and the dispensing nozzle 45 approaches the crystallization plate 16. Then the lower end of the dispensing tip or nozzle stops at a predetermined height corresponding to the object for the dispensing.

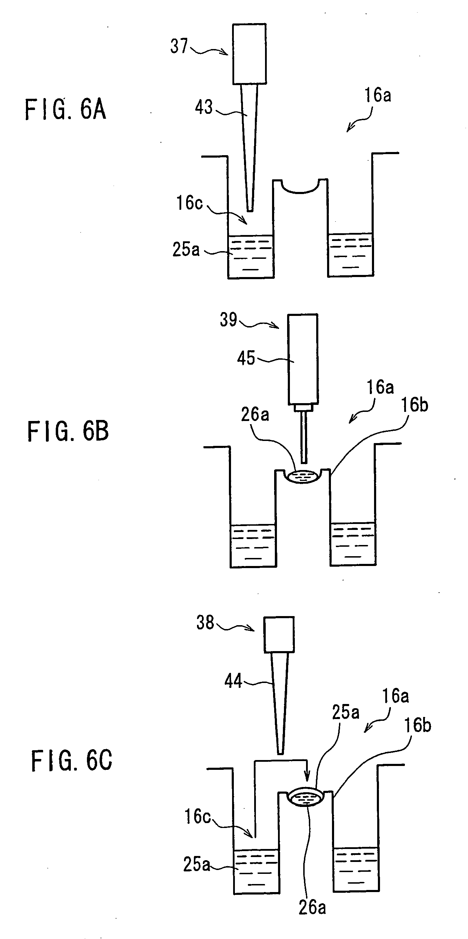

FIG. 6 shows an example of the dispensing operation to be performed in the operation of preparing a crystallization plate to be carried out using this dispensing head part 33. First, the first dispensing head 37 is selected. Then the crystallization solution 25a is drawn from the crystallization solution reservoir 25 into the first dispensing tip 43. Subsequently, as shown in FIG. 6A, the crystallization solution 25a is dispensed in the reservoir 16c of the well 16a with the first dispensing tip 43. In this case, since the first dispensing tip 43 is a large-sized tip for the crystallization solution, the dispensing can be completed in a short period of time even when a large amount of crystallization solution 25a is to be dispensed in the reservoir 16c.

Thereafter, the third dispensing head 39 is selected to draw the protein solution 26a from the protein solution reservoir 26. Subsequently, as shown in FIG. 6B, with respect to the well 6a having the reservoir 16c in which the crystallization solution 25a has been dispensed, the protein solution 26a is dispensed in the pocket located at the top of the liquid holding part 16b.

Next, the second dispensing head 38 is selected. Then, as shown in FIG. 6C, part of the crystallization solution 25a contained in the reservoir 16c is drawn in with the second dispensing tip 44. Subsequently, a predetermined amount of crystallization solution 25a is added to and is mixed with the protein solution 26a that already has been dispensed in the liquid holding part 16b. In this case, since the second dispensing tip 44 is a small-sized tip for a crystallization solution, the dispensing can be carried out with high accuracy even when a trace amount of crystallization solution is to be added to and to be mixed with the protein solution 26a. Furthermore, the mixture ratio between the protein solution 26a and the crystallization solution 25a to be mixed together in the liquid holding part 16b can be set arbitrarily using the second dispensing tip, with various combinations of the protein solution 26a and the crystallization solution 25a being employed. As a result, the protein solution can be adjusted to have various concentrations. Hence, screening can be carried out easily, with the protein concentration conditions being varied, without preparing protein solutions with various concentrations beforehand.

In the above-mentioned configuration, the dispensing head part 33 and the aforementioned dispensing-head moving mechanism compose a dispensing means that dispenses the crystallization solution 25a and the protein solution 26a in the reservoir 16c and the liquid holding part 16b, respectively, with respect to the well 16a of the crystallization plate 16 that has been set on the dispensing stage 11a. This dispensing means is configured to have the single dispensing head part 33 that is provided with a crystallization solution dispensing head (the third dispensing head 39) that dispenses the crystallization solution 25a and a protein solution dispensing head that dispenses the protein solution 26a.

Furthermore, the first dispensing head 37 that dispenses the crystallization solution 25a in the reservoir 16c and the second dispensing head 38 that dispenses the crystallization solution 25a in the liquid holding part 16b are provided to serve as the above-mentioned protein solution dispensing head. In the aforementioned dispensing operation (see FIG. 6), the second dispensing head 38 draws in the crystallization solution 25a from the reservoir 16c and then dispenses it in the liquid holding part 16b.

The following description is directed to a seal attachment unit 50 that is provided on one side (on the side opposite to the stock section 12) with respect to the dispensing stage 11a shown in FIG. 2 as an example of the sealing means. However, the sealing means of the present invention is not limited to the seal attachment unit but can be a sealing means employing thermocompression bonding or the like, for instance. A slide table 51 is provided on the operation area 11. A plate holding part 52 that holds the crystallization plate 16 is attached to the slide table 51 so as to be slidable in the Y direction. The plate holding part 52 is reciprocated in the Y direction by a moving means (not shown in the drawings). A crystallization plate 16 that has been subjected to the dispensing operation completed on the dispensing stage 11a is placed on the plate holding part 52 to be held thereby.

A seal attachment head 55 is disposed above the slide table 51 so as to move up and down. A sheet-like seal member 56 drawn out from a seal feed part 53 is fed to the seal attachment head 55. The seal member 56 is fed, with a peelable paper being attached thereto. In the seal attachment operation, the crystallization plate 16 is moved relatively to the horizontal direction (i.e. the Y direction) with respect to the seal attachment head 55, with the seal attachment head 55 pressing the upper surface of the crystallization plate 16 held by the plate holding part 52.

Thus the seal member 56 is attached to the upper surface of the crystallization plate 16. The peelable paper detached from the seal member 56 then is wound up to be collected by a peelable paper collecting part 54. This attachment of the seal member 56 allows the upper surfaces of all the wells 16a to be covered and sealed (see FIG. 3). The seal attachment unit 50 is a sealing means that seals the wells 16a in which the crystallization solution 25a and the protein solution 26a have been dispensed.

One side of the housing 3 is provided with an opening 3c for carrying out the crystallization plate 16, in a place adjoining the seal attachment unit 50. The crystallization plate 16 with the seal member 56 attached to the upper surface thereof by the seal attachment unit 50 is carried into the protein crystal detection apparatus 5 through the opening 3c by the transfer head 21. A bar code reader 57 is provided for the opening 3c. The ID code given to the crystallization plate 16 to be carried out is read by the bar code reader 57. This allows each crystallization plate 16 to be identified. The seal member is not particularly limited but is preferably a transparent film that tends not to be elastic. Examples of the film include a film of polyolefin, specifically, a 3M tape 9795 (trade name) manufactured by 3M, etc.

Next, an example of the configuration of a control system of the apparatus for preparing a protein crystallization plate is described with reference to FIG. 7. In FIG. 7, the apparatus 2 for preparing a protein crystallization plate has a communication function and is connected to a host computer 67 that is a host controller through a LAN system 66 connected to a communication interface 65. The communication interface 65 is connected to a processing unit 60.

The processing unit 60 runs various processing programs stored in a program memory unit 62 according to various data stored in a data memory unit 61 and thereby performs various operations and processing functions to be described later. In this case, an operation program 62a for preparing a protein crystallization plate has been stored in the program memory unit 62. When this program is executed, the operation for preparing a protein crystallization plate is performed.

The data memory unit 61 includes a consumable-information memory part 61a, a dispensation-operation-information memory part 61b, a supply-reservoir-information memory part 61c, and a crystallization-plate-information memory part 61d. The consumable-information memory part 61a is allowed to store information about consumables to be used in the operation for preparing a protein crystallization plate, i.e. information about stocks of the crystallization plates 16 and the tip racks 17 and 18 supplied to the stock section 12 as well as the seal member 56 supplied to the seal feed part 53.

This stock information includes the positions where they are stocked and the quantity of stocks remaining at each timing during the operation of the apparatus. The transfer part 20 takes out the respective consumables from the stock section 12 according to this stock information. The quantity of the stock is updated in real time by subtracting the quantity of consumed items from the initial value of the teaching input that has been input beforehand, every time the dispensing operation is carried out. This continuous monitoring of the stock quantity makes it possible to prevent the apparatus from stopping due to running out of the consumables or to report it beforehand.

The dispensing-operation-information memory part 61b stores information about the dispensing operation that is downloaded from the host computer 67, that is, information required for dispensing a predetermined amount of a predetermined liquid in a predetermined well 6a of the crystallization plate 16 using the dispensing means. This dispensing operation information includes well information indicating the positions of the wells 16a arranged in the crystallization plate 16 and information indicating the combination of the protein solution 26a and the crystallization solution 25a (i.e. which kinds of protein solution 26a and crystallization solution 25a are to be dispensed in one well).

The supply-reservoir-information memory part 61c stores supply reservoir information, i.e. information indicating positions in the operation area 11 of the protein solution supply reservoir 26 and the crystallization solution supply reservoir 25 as well as the kinds of the solutions stored in the respective wells of these reservoirs. The dispensing head driving mechanism operates according to the above-mentioned dispensing operation information and the supply reservoir information to allow a predetermined solution to be drawn correctly with the dispensing head part 33.

The crystallization-plate-information memory part 61d stores crystallization plate information in which with respect to each crystallization plate 16 that has been subjected to the dispensing operation, the crystallization plate and the dispensing operation information are related to each other. That is, the crystallization-plate-information memory part 61d stores the information that makes it possible to identify the combination of the crystallization solution 25a and the protein solution 26a that have been dispensed in one well with respect to each well 16a of the individual crystallization plate 16 that is distinguished/identified by its ID code. This makes it possible to match the protein crystal detection results with the crystallization conditions (i.e. the combination of the protein solution 26a and the crystallization solution 25a) correctly in observing the protein crystallization after the dispensing operation.

The processing unit 60 controls the operations of the dispensing-head moving mechanism 30, the dispensing head part 33, the stock section 12, the transfer part 20, and the seal attachment unit 50 according to a program 62a of the operation for preparing a protein crystallization plate. The bar code reader 57 transmits the ID code read from the crystallization plate 16 to be carried out through the opening 3c to the processing unit 60. With this operation, the ID code to be used for preparing the crystallization plate information described above is provided. A display processing part 63 carries out a process for allowing a display unit to display a guidance picture at the time of data input, for example. An operation/input processing part 64 gives operational commands and carries out data input with respect to the processing unit 60 using an input means such as touch keys provided for the control panel 4.

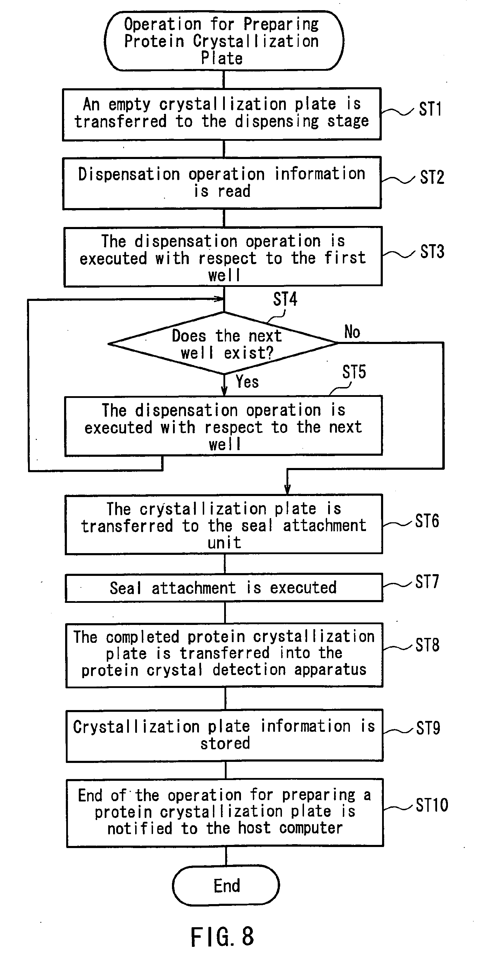

Next, an example of the operation for preparing a protein crystallization plate is described with reference to FIG. 8. This operation is carried out when the processing unit 60 executes the program 62a of the operation for preparing a protein crystallization plate. With this operation, a crystallization plate is prepared in which a protein solution and a crystallization solution have been dispensed in each well in order to screen conditions for protein crystallization by the vapor diffusion method.

First, with the transfer part 20, an empty crystallization plate 16 is taken out from the stock section 12 and then is transferred to the dispensing stage 11a (ST1). Subsequently, the dispensing operation information is read from the dispensing-operation-information memory part 61b (ST2). The dispensing head part 33 is moved with respect to the crystallization plate 16 according to the dispensing operation information and then the dispensing operation is executed with respect to the first well 16a (ST3). Thus, the dispensing operation shown in FIG. 6 is executed.

Thereafter, the presence of the next well is judged (ST4). When the next well exists, the dispensing operation is executed with respect to the next well (ST5). Thereafter, the same process is executed repeatedly until it is judged in the step ST4 that the next well does not exist. When it is judged in the step ST4 that the next well does not exist, the crystallization plate 16 that has been subjected to the dispensing operation is transferred to the seal attachment unit 50 (ST6).

In the seal attachment unit 50, a seal is attached to the upper surface of the crystallization plate 16 (ST7). Then the completed crystallization plate 16 is transferred to the protein crystal detection apparatus 5 (ST8). The ID code of the crystallization plate 16 that is read therefrom during the transfer is combined with the dispensing operation information and thereby crystallization plate information is produced and is stored in the crystallization-plate-information memory part 61d (ST9). Thereafter, the end of the operation for preparing a protein crystallization plate is notified to the host computer 67. Thus all the operations are completed.

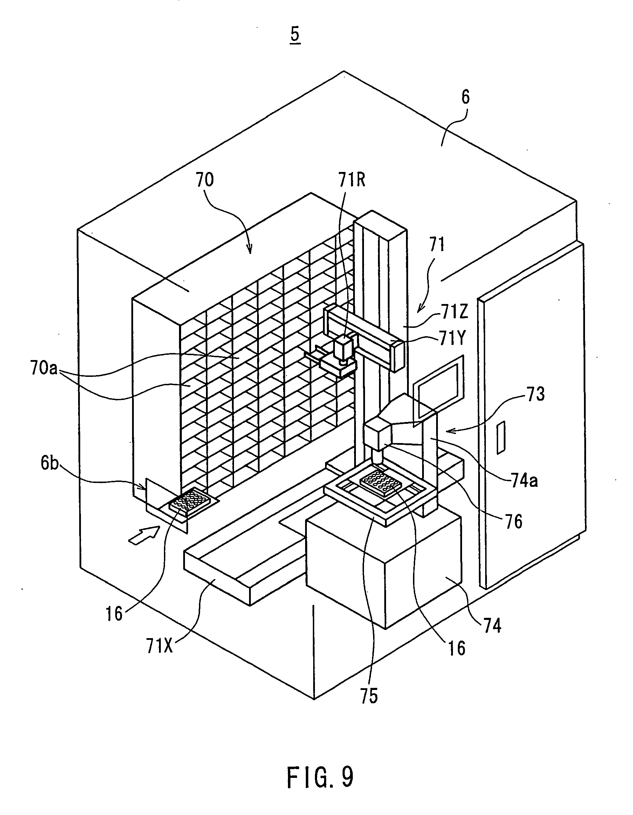

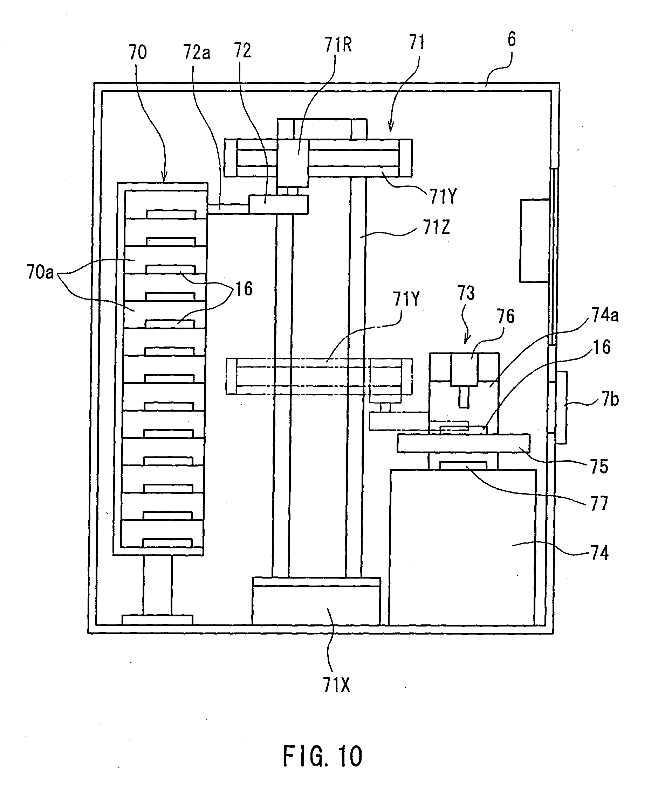

Next, an example of the overall configuration of the protein crystal detection apparatus 5 is described with reference to FIGs. 9 and 10. The protein crystal observation apparatus 5 is used for detecting protein crystals generated in the protein solution by the vapor diffusion method with respect to the crystallization plate 16 prepared with the apparatus 2 for preparing a protein crystallization plate.

In FIGs. 9 and 10, the protein crystal detection apparatus 5 is configured with a storage unit 70, a transfer unit 71, and an observation section 73 that are disposed in a thermostatic chamber formed in a box-shaped housing 6. The thermostatic chamber 6 is provided with a temperature adjustment function for maintaining the inner atmosphere in a predetermined environment. As shown in FIG. 9, the housing 6 has one side that is provided with a delivery door 6b. The delivery door 6b is used for delivering, into the housing 6, the crystallization plate 16 that is carried out through the opening 3c of the apparatus 2 for preparing a protein crystallization plate. The delivery door 6b can be opened and closed with a delivery door opening/closing mechanism (not shown in the drawings).

The following description is directed to the internal configuration of the thermostatic chamber. Inside the thermostatic chamber, the vertically placed, shelf-shaped storage unit 70 is disposed along the back-side wall surface. The storage unit 70 includes a plurality of storage parts 70a, into which the storage unit 70 is divided in the form of a shelf. Each storage part 70a stores one crystallization plate 16 that has been subjected to the dispensing operation in the apparatus 2 for preparing a crystallization plate and has wells 16a that have been sealed. The protein crystal detection apparatus 5 is a crystallization vessel storage means that stores a plurality of the crystallization plates 16 whose wells 16a have been sealed, in a predetermined environment.

The transfer unit 71 is disposed in front of the storage unit 70. The transfer unit 71 includes an X table 71X, a Y table 71Y, a Z table 71Z, a rotation head 71R, and a plate holding head 72. The X table 71X is provided horizontally on the floor surface in the X direction (in the direction parallel with the storage unit 70). The Y table 71Y is attached to the Z table 71Z attached to the X table 71X in a standing state so as to be placed horizontally. Furthermore, the rotation head 71R is attached to the Y table 71.

The plate holding head 72 is attached to the rotation axis of the rotation head 71R. Driving the X table 71X, the Y table 71Y, and the Z table 71Z allows the plate holding head 72 to move in the X, Y, and Z directions in front of the storage unit 70. In addition, driving the rotation head 71R allows the horizontal orientation of the holding head 8 to be changed.

The movements in the X, Y, and Z directions of the plate holding head 72 allow an arm 72a to catch and hold the plate 16 delivered through the delivery door 6b and to place the plate 16 in a designated storage part 70a of the storage unit 70. The crystallization plate 16 that has been kept for a predetermined period of time in the storage part 5a is held by the plate holding head 72 of the transfer unit 71 and the movement of the plate holding head 72 allows it to be transferred to the observation section 73.

The observation section 73 is configured as follows. That is, an observation table 75 is attached horizontally to a frame 74a attached to a base 74 in a standing state, and a camera 76 is placed above the observation table 75. A crystallization plate 16 transferred by the plate holding head 72 is placed and set on the observation table 75. Driving the XYZ moving mechanism provided for the observation table 75 allows the plate 16 to move in the X, Y, and Z directions.

The crystallization plate 16 is stored in this state in a predetermined temperature atmosphere and thereby a solvent component contained in the protein solution 26a is evaporated. Accordingly, the protein concentration of the protein solution 26a increases, which brings the protein solution 26a into a supersaturation state to produce protein crystals. In this case, the evaporation of the solvent from the protein solution 26a progresses gradually, with the solvent that evaporates from the protein solution 26a being kept in equilibrium with the vapor to be absorbed by the crystallization solution 25a. Thus, crystals are produced stably.

In the observation section 73, the crystallization plate 16 is observed during such a crystal production process and thereby the presence of protein crystals and the degree of crystallization in each well 16a are detected. That is, the observation function of the observation section 73 and the processing function to be achieved with a crystal detection program 82a executed by a processing unit 80 to be described later serve as a protein crystal detection means for detecting protein crystals produced in the protein solution 26a contained in the sealed well 16a.

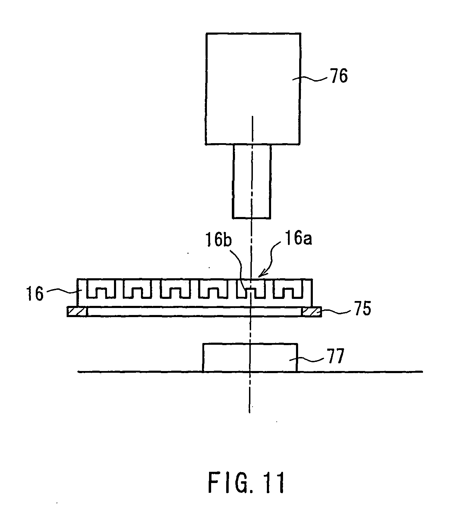

FIG. 11 shows an example of the observation operation for capturing an observed image of the protein solution. As shown in FIG. 11, the crystallization plate 16 set on the observation table 75 is moved to a position below the camera 76. Then the liquid holding part 16b provided in the well 16a to be observed is aligned with an image-pickup optical axis of the camera 76. Thereafter, the image of the crystallization plate 16 is picked up with the camera 76, with the crystallization plate 16 being irradiated with light emitted from a lighting unit 77 disposed below. Thus, the observed image of the protein solution contained in the crystallization plate 16 is captured.

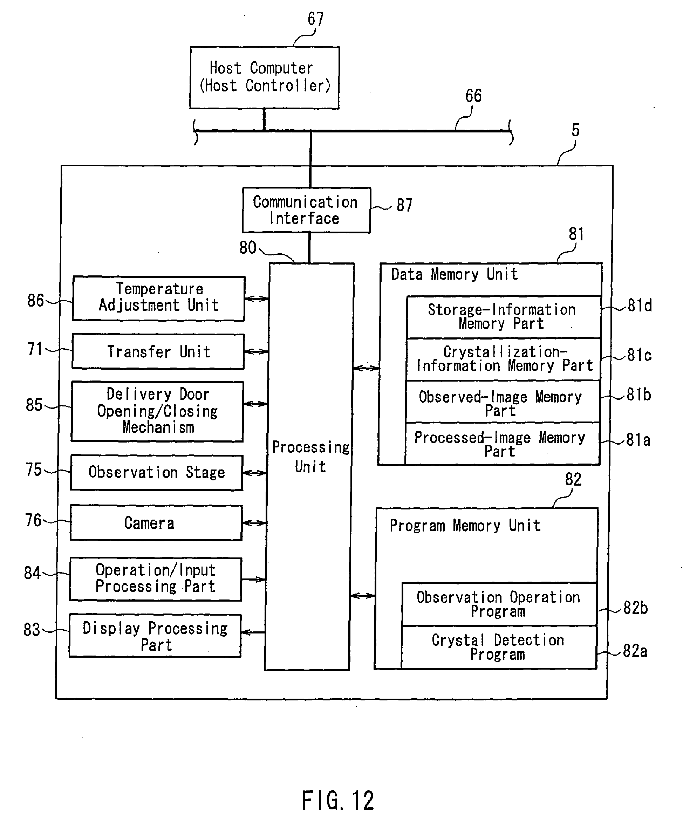

Next, an example of the configuration of a control system of a protein crystal observation apparatus is described with reference to FIG. 12. In FIG. 12, the protein crystal detection apparatus 5 has a communication interface 87. The communication interface 87 transfers control signals between a processing unit 80 that performs control processing in the protein crystal detection apparatus 5 and the host computer 67 that is a host controller, through a LAN system 66.

The processing unit 80 executes various processing programs stored in a program memory unit 82 according to the various data stored in a data memory unit 81 and thereby implements the various operations and processing functions to be described later. In this case, a crystal detection program 82a and an observation operation program 82b are stored in the program memory unit 82. The protein solution observation operation and the process for detecting protein crystals contained in the protein solution, which are to be described later, are performed through the execution of those programs.

The data memory unit 81 includes a processed-image memory part 81a, an observed-image memory part 81b, and a crystallization-information memory part 81c. The processed-image memory part 81a stores processed images that have been subjected to various processes in the protein crystal detection process. The observed-image memory part 81b stores observed images of the protein solution 26a captured with the camera 76.

In the protein crystal detection process to be described later, the observed images stored in the observed-image memory part 81b are objects to be processed. The crystallization-information memory part 81c stores crystallization information, i.e. image data of the observed images with which crystallization was detected in the protein crystal detection process, information that identifies the crystallization plate and well whose observed image has been obtained, and information about an observation time at which the crystallization plate concerned was observed, for example.

Furthermore, a display processing part 23, an operation/input processing part 24, a camera 76, an observation stage 75, a delivery door opening/closing mechanism 85, a transfer unit 71, and a temperature adjustment unit 86 are connected to the processing unit 80. The temperature adjustment unit 86 adjusts the inner temperature of the thermostatic chamber according to the temperature command delivered through the processing unit 80 from the host computer 67. Thus the inner temperature of the thermostatic chamber is maintained at a preset temperature.

The transfer unit 71 performs operations for transferring the crystallization plate 16 within the thermostatic chamber 6, i.e. transferring operations such as an operation for storing the plate 16, in a predetermined storage part 70a of the storage unit 70, which is carried in through the delivery door 6b provided for the thermostatic chamber 6, as well as an operation for taking out the crystallization plate 16 from the storage part 70a to set it in the observation section 73 according to the control signals sent from the processing unit 80. The delivery-door opening/closing mechanism 85 opens and closes the delivery door 6b according to the control signals sent from the processing unit 80.

Furthermore, the processing unit 80 controls the observation table 75 and the camera 76, and thereby the crystallization plate 16 held by the observation table 75 is moved and the image of the protein solution is captured with the camera 76. The display processing part 83 displays observed images captured with the camera 76 and various processed images, and also performs a process for displaying, for example, guidance pictures in inputting data. The operation/input processing part 84 gives operational commands and carries out data inputs with respect to the processing unit 80 through the operation of an input device such as a keyboard.

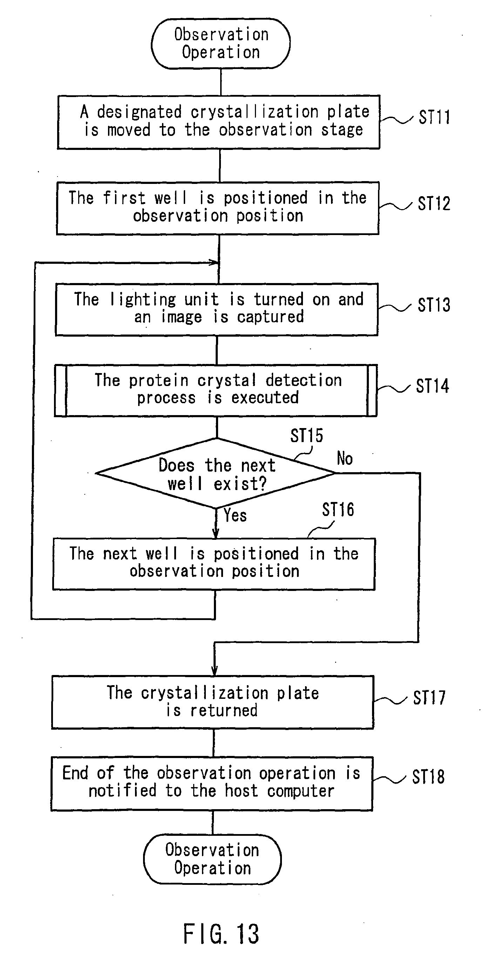

Next, an example of the observation operation for detecting protein crystals is described with reference to the flow chart shown in FIG. 13. This observation operation is carried out, with the processing unit 80 executing the observation operation program 82b. By the time the observation operation is to be started, the crystallization plate 16 carried in through the delivery door 6b from the apparatus located on the upstream side has been received by and stored in the storage part 70a.

First, in FIG. 13, a designated crystallization plate 16 is taken out by the transfer unit 71 to be moved to the observation stage 75 (ST11). Then the first well 16a is positioned in the observation position directly under the camera 76 (ST12). Thereafter, the lighting unit 77 is turned on and an image is captured with the camera 76 (ST13). Subsequently, the protein crystal detection process to be described later is carried out (ST14).

When the protein crystal detection process is completed with respect to this well, it is judged whether the next well exists (ST15). In the case where the next well exists, the next well is positioned in the observation position (ST16), and the procedure returns to the step ST13 and the same process is executed repeatedly. When it is judged in the step ST15 that the next well does not exist, the crystallization plate 16 whose process has been completed is returned to the storage part 70a (ST17). Then the end of the observation operation is notified to the host computer 67 (ST18) and thus the observation operation execution process is completed.

In the above-mentioned observation operation, all the operations can be carried out without taking the crystallization plate 16 to the outside of the thermostatic chamber. As compared to the system in which the crystallization plate is taken out when it is to be observed, variations in crystal growth conditions caused by the change in temperature condition of the crystallization plate can be eliminated, which allows screening accuracy to be improved. In addition, fog resulting from dew condensation that occurs when the crystallization plate in a cooled state is exposed to room temperature dose not form in the observation field of view, and thus excellent observation results can be obtained. Accordingly, the observation operation for detecting protein crystals can be performed efficiently with high reliability.

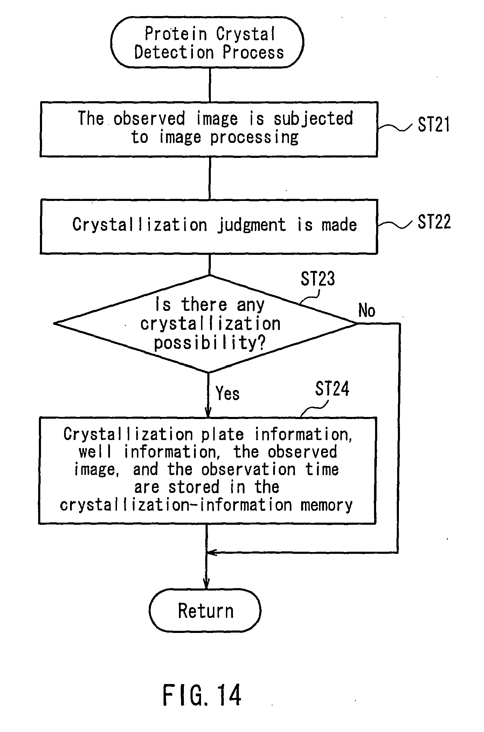

Next, an example of the protein crystal detection process that is executed in the step ST14 is described with reference to FIG. 14. First, an observed image is subjected to image processing (ST21), and then crystallization judgment is made (ST22). If it is judged that there is no crystallization possibility, the process ends. On the other hand, if it is judged in the step ST 22 that there is a crystallization possibility, crystallization information is stored in the crystallization-information memory part 21c (ST24), wherein the crystallization information includes the crystallization plate information that indicates the crystallization plate 6 subjected to this process from which the observed image was obtained, the well information, the observed image, the observation time, etc. Thus the protein detection process ends with respect to the observed image concerned.

For the judgment of crystallization, for instance, a combination of conventional image processing techniques may be used or a system may be developed especially for the judgment of crystallization.

In the configuration of the apparatus 1 for screening protein crystallization conditions in which the above-mentioned apparatus 2 for preparing a crystallization plate and the protein crystal detection apparatus 5 are combined together, the transfer part 20 of the apparatus 2 for preparing a crystallization plate is a first transfer means with respect to the dispensing stage 11a and the sealing means while the transfer unit 71 of the protein crystal detection apparatus 5 is a second transfer means with respect to the crystallization solution storage means. The first transfer means and the second transfer means compose the crystallization vessel transfer means.

The protein crystal detection apparatus 5 is provided with: a thermostatic chamber that stores, in a predetermined environment, a plurality of crystallization plates 16 whose wells 16a have been sealed; and the observation section 73 where protein crystals are detected that have been produced in the crystallization plates 16 placed in the thermostatic chamber. The above-mentioned second transfer means is configured to transfer the crystallization plates 16 within the thermostatic chamber.

With the above-mentioned configuration, crystallization plates can be prepared automatically with high efficiency by the vapor diffusion method using the sitting drop technique. Thus, screening of protein crystallization conditions can be carried out efficiently by the vapor diffusion method using the sitting drop technique. Furthermore, it also can be used for the production of protein crystals, for example.

In the above-mentioned embodiment, an example was described in which the observation section 73 that serves as a protein crystal detection means is placed in the thermostatic chamber that stores the crystallization plates 16. However, the observation section may be provided outside the thermostatic chamber. Moreover, the system was described in which the protein solution contained in the crystallization plate 16 was observed with the camera 10 that serves as a protein crystal detection means. However, protein crystals may be detected using other methods.

### Industrial Applicability

With the screening apparatus of the present invention, the protein crystallization conditions can be screened efficiently by the vapor diffusion method using the sitting drop technique. Furthermore, the screening apparatus of the present invention also can be used for the production of protein crystals, etc. in addition to the screening of the protein crystallization conditions.

## Claims

1. An apparatus for screening protein crystallization conditions that screens protein crystallization conditions using a sitting drop technique that is one of techniques of protein crystallization to be carried out by a vapor diffusion method, the apparatus comprising:
a dispensing stage where a crystallization vessel is set, the crystallization vessel being provided with a plurality of solution storage parts, each solution storage part including a solution holding part that holds a protein solution in a seating state from a lower side and a reservoir that retains a crystallization solution;
a dispensing means that dispenses the crystallization solution in the reservoir and dispenses the protein solution in the solution holding part, in the solution storage parts of the crystallization vessel set on the dispensing stage;
a sealing means that seals the solution storage parts in which the crystallization solution and the protein solution have been dispensed;
a crystallization vessel storage means that stores a plurality of crystallization vessels in a predetermined environment, the solution storage parts of each of the crystallization vessels having been sealed;
a protein crystal detection means that detects protein crystals produced in the protein solution contained in the solution storage parts that have been sealed; and
a crystallization vessel transfer means that transfers the crystallization vessel to at least one of the dispensing stage, the sealing means, the crystallization vessel storage means, and the protein crystal detection means.

2. The apparatus for screening protein crystallization conditions according to claim 1, wherein the dispensing means comprises a crystallization solution dispensing head that dispenses the crystallization solution and a protein solution dispensing head that dispenses the protein solution.

3. The apparatus for screening protein crystallization conditions according to claim 2, wherein the crystallization solution dispensing head comprises a first dispensing head that dispenses the crystallization solution in the reservoir and a second dispensing head that dispenses the crystallization solution in the solution holding part.

4. The apparatus for screening protein crystallization conditions according to claim 3, wherein the second dispensing head draws in the crystallization solution from the reservoir and then dispenses it in the solution holding part.

5. The apparatus for screening protein crystallization conditions according to claim 2, wherein the dispensing means comprises: a single dispensing head part including the crystallization solution dispensing head and the protein solution dispensing head; and a dispensing head moving means that moves the dispensing head part with respect to the dispensing stage.

6. The apparatus for screening protein crystallization conditions according to claim 3 or 4, wherein the dispensing means comprises: a single dispensing head part including the first dispensing head, the second dispensing head, and the protein solution dispensing head; and a dispensing head moving means that moves the dispensing head part with respect to the dispensing stage.

7. The apparatus for screening protein crystallization conditions according to claim 1, wherein the crystallization vessel transfer means comprises: a first transfer means that transfers the crystallization vessel to at least one of the dispensing stage and the sealing means; and a second transfer means that transfers the crystallization vessel to the crystallization solution storage means.

8. The apparatus for screening protein crystallization conditions according to claim 7, wherein the crystallization vessel storage means comprises: a thermostatic chamber in which the crystallization vessel is stored in the predetermined environment; and a protein crystal detection means that detects crystals of protein produced in the crystallization vessel placed in the thermostatic chamber, and the second transfer means transfers the crystallization vessel within the thermostatic chamber.
